# EUROPEAN PATENT APPLICATION

(11) **EP 1 958 576 A2**
(43) Date of publication of application: **20.08.2008**
(21) Application number: 08101521.6
(22) Date of filing: 12.02.2008
(51) Int. Cl.: A61B 17/32

(54) **Double cut shaver**

(30) Priority: 13.02.2007 US 900991 P
(71) Applicant: Arthrex Inc, Naples, FL 34108-1945 (US)
(72) Inventor: Bickenbach, Christine, Naples, FL 34119-9524 (US)
(74) Representative: Strandin, Heléne

(57) **Abstract**

A surgical cutting instrument with high resection efficiency due to advanced cutting edge tooth geometry. The cutting instrument comprises a tubular member with at least two cutting windows, preferably two cutting windows, provided at the distal end of the tubular member. The two cutting windows have one most distal common region, preferably provided about perpendicular to the longitudinal axis of the tubular member. At least one of the two cutting windows may be provided with teeth. The two cutting windows may be symmetrically or asymmetrically disposed relative to the tube axis when viewed in a plan view, independent of the teeth orientation.

## Description

### FIELD OF THE INVENTION

The present invention is directed to surgical cutting instruments used in arthroscopic and endoscopic surgery.

### BACKGROUND OF THE INVENTION

Surgical cutting instruments in which an inner member is rotated within an elongate tubular outer member are known in surgical procedures where access to the surgical site is via a narrow portal or passage. Typically, the tubular outer member has a distal end with an opening defining a cutting port or window. The inner member has a distal end with a cutting tip for engaging bodily tissue via the opening. Proximal ends of the inner and outer members commonly include hubs which attach to a handpiece having a motor for rotating the inner member relative to the outer member. The distal end of the inner member can have various configurations dependent upon the surgical procedure to be performed. Often the inner member is tubular so that the loose tissue resulting from a cutting, resecting or abrading procedure can be aspirated through the lumen of the inner member.

The cutting port or window of the tubular outer member is typically provided with teeth having angled configurations and being fabricated by a two-step process. First, the profile of the teeth is formed in the conventional through-cut manner. Subsequently, the teeth are "sharpened" by removing material by Electrical Discharge Machining (EDM), to form a beveled surface on the portion of a tooth in contact with the tube inner lumen. The resulting teeth are effective for penetrating tissue and preventing ejection of tissue from the cutting window as the inner and outer cutting edges approach each other. However, this two-step approach has several drawbacks. Although EDM is used to remove the material to form the beveled surface (as conventional machining processes are unable to produce the required geometry under production conditions), the EDM process has nevertheless high consumable tooling costs as the electrode is eroded during use. In addition, because EDM removes material by melting and vaporization, it is difficult to produce a sharp point on the teeth. The surfaces produced by EDM are also generally rough and present a high resistance to tissue sliding over the surface, inhibiting penetration of teeth into tissue.

Accordingly, there is a need to provide an improved cutting instrument used in arthroscopic surgery, that has a sharper cutting edge and that is produced with more control over the angle of the teeth during manufacturing. A surgical cutting instrument that has sharper cutting edges with cutting teeth at varying angles is also needed. A method of fabricating cutting edges for cutting instruments, such as shaver blades, with high resection efficiency due to advanced cutting edge tooth geometry is also needed.

### SUMMARY OF THE INVENTION

The present invention provides a surgical cutting instrument with high resection efficiency due to advanced cutting edge tooth geometry. The cutting instrument comprises a tubular member with at least two cutting windows, preferably two cutting windows, provided at the distal end of the tubular member. The two cutting windows have one most distal common region, preferably provided about perpendicular to the longitudinal axis of the tubular member. At least one of the two cutting windows may be provided with teeth. The two cutting windows may be symmetrically or asymmetrically disposed relative to the tube axis when viewed in a plan view, independent of the teeth orientation. The teeth of the cutting instrument are advantageously formed with a laser, rather than EDM, resulting in very sharp teeth.

These and other features and advantages of the invention will be more apparent from the following detailed description that is provided in connection with the accompanying drawings and illustrated exemplary embodiments of the invention.

### BRIEF DESCRIPTION OF THE DRAWINGS

FIG. 1 is a top view of the distal end of a double-window tubular member of the surgical cutting instrument of the present invention;

FIG. 2 is a perspective view of the distal end of the double-window tubular member of FIG. 1;

FIG. 3 is another perspective view of the distal end of the double-window tubular member of FIG. 1;

FIG. 4 is another perspective view of the distal end of a double-window tubular member of the present invention and according to another embodiment; and

FIG. 5 illustrates the sharper edge that can be obtained with laser cutting as compared to EDM cutting.

### DETAILED DESCRIPTION OF THE PREFERRED EMBODIMENTS

The following description is provided to enable any person skilled in the art to make and use the invention and sets forth the best modes contemplated by the inventors of carrying out their invention. Various modifications, however, will remain readily apparent to those skilled in the art.

The present invention provides a surgical cutting instrument with high resection efficiency due to advanced cutting edge tooth geometry. The cutting instrument comprises a tubular member with at least two cutting windows, preferably two cutting windows, provided at the distal end of the tubular member. Each of the cutting windows has a plurality of teeth positioned along lateral cutting edges, the teeth being configured for easy penetration into tissue to prevent ejection of tissue from the cutting windows during closure. At least one of the cutting edges is formed by laser cutting technology. Preferably, both cutting edges are formed by laser cutting technology.

The two cutting windows have one most distal common region, preferably provided about perpendicular to the longitudinal axis of the tubular member. In one embodiment, the teeth of each window are symmetrically disposed relative to the tube axis when viewed in a plan view. In another embodiment, the teeth are asymmetrically disposed. The two cutting windows may be symmetrically or asymmetrically disposed relative to the tube axis when viewed in a plan view, independent of the teeth orientation.

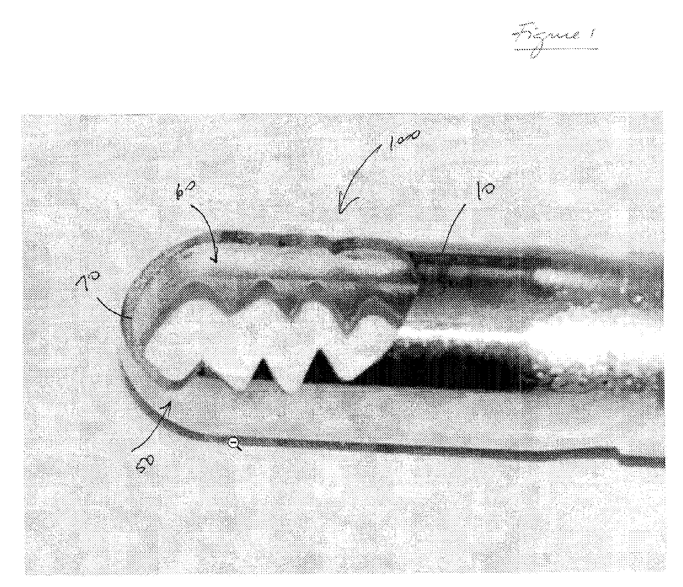

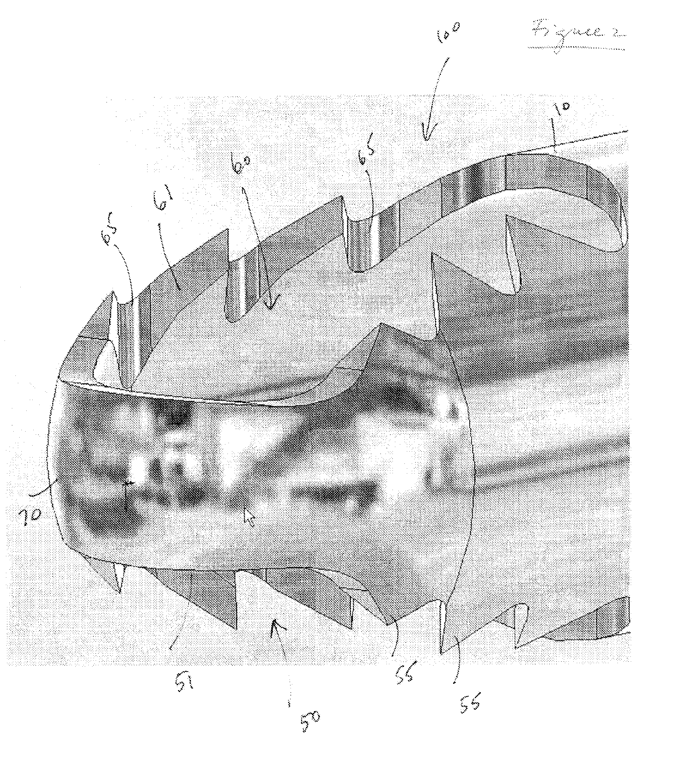

FIGS. 1-4 illustrate exemplary embodiments of tubular member 100 of a surgical cutting instrument of the present invention, which is provided with two cutting windows 50, 60, at least one of the cutting windows being manufactured by conducting a laser cutting process.

As shown in the drawings, tubular member 100 comprises a distal end portion 10 that includes cutting means including at least two cutting windows 50, 60 having a plurality of teeth 55, 65 positioned along lateral cutting edges 51, 61, the teeth being configured for easy penetration into tissue to prevent ejection of tissue from the cutting windows during closure. At least one of the cutting edges 51, 61 is formed by laser cutting technology. Preferably, both cutting edges 51, 61 are formed by laser cutting technology.

As also shown in FIGS. 1-4, the two cutting windows 50, 60 have a most distal common region 70, with an exemplary curved or arcuate configuration and preferably oriented about perpendicular to longitudinal axis 11 of the tubular member. Common region 70 may have, however, any geometry and configuration (for example, semicircular or parabolic configuration) which allows it to act as a "bridge" between the two cutting windows, i.e., providing a "closed mouth" at the distal end of the instrument 100. In this manner, the cutting instrument is stronger than the conventional "open mouth" EDM instruments (which necessitate the "open mouth" configuration to allow the wire to enter and exit the distal end of the instrument during EDM cutting of the teeth).

In one embodiment, the teeth of each window may be symmetrically disposed relative to the tube axis 11 when viewed in a plan view. In another embodiment, the teeth may be asymmetrically disposed. In other embodiments, the two cutting windows 50, 60 may be symmetrically disposed relative to the tube axis 11 when viewed in a plan view. Alternatively, the two cutting windows 50, 60 may be asymmetrically disposed relative to the tube axis 11 when viewed in a plan view.

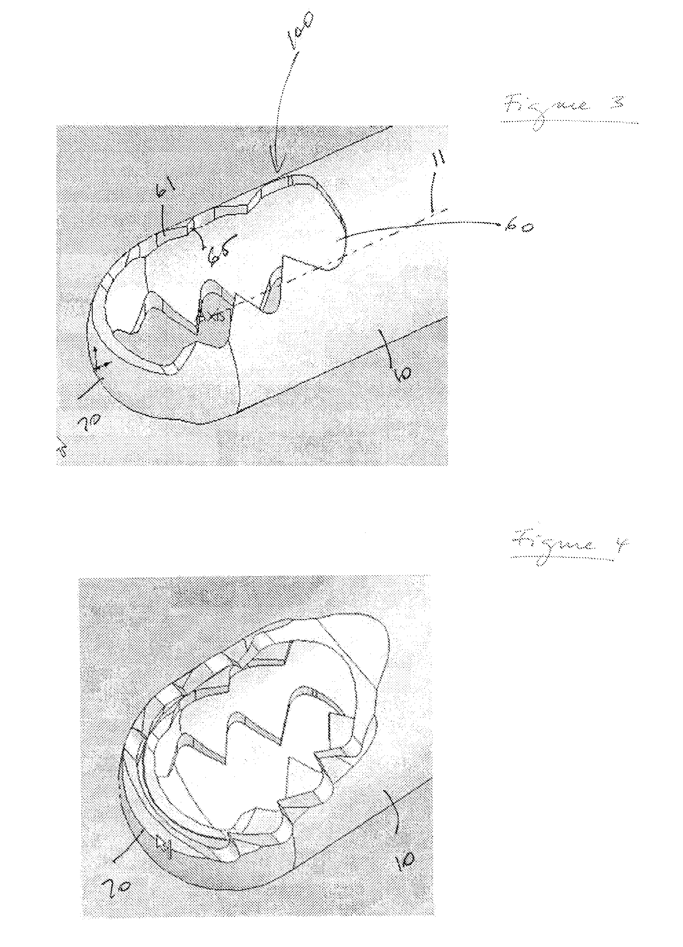

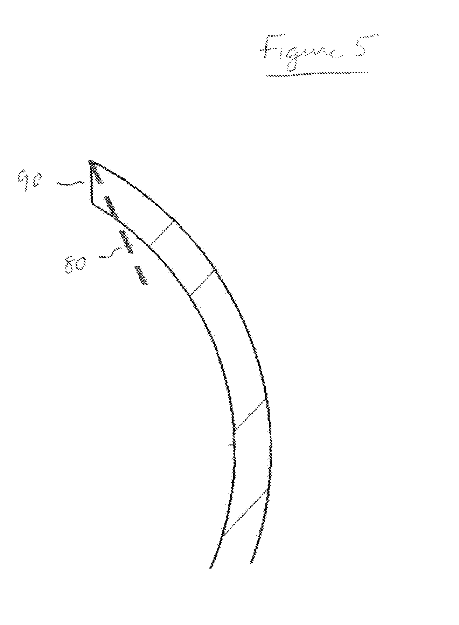

According to embodiments of the present invention, the step of cutting the teeth 55, 65 with laser is conducted by cutting from top down (i.e., from top to bottom), and not from the side profile (as in conventional wire EDM technology). The top-down orientation affords a "sharper" cutting edge, since the teeth are not cut straight across but rather from the top, making the beveled edge much sharper. Another advantage of the laser cutting technology is that there is more control over the laser angles as compared to the wire EDM, affording more control during manufacturing. Wire EDM does not allow any varying of the angle of the teeth during cutting. In contrast, by employing the laser technology to form the teeth, the angle can be changed to obtain a sharper cutting edge on the teeth. FIG. 4 illustrates, for example, various angles on the teeth (in contrast with the teeth of the prior art that are straight across the top, which afford a less sharper edge). FIG. 5 illustrates the sharper edge that can be obtained with laser cutting 80 as compared to standard EDM cutting 90.

Tubular member 100 of the present invention may be an inner member or an outer member of a cutting instrument, for example, an arthroscopic shaver, and may be disposed coaxially or concentrically within a corresponding outer or inner tube. Tubular member 100 may be formed from a medically acceptable material such as stainless steel. In a preferred embodiment, tubular member 100 has a hollow cylindrical configuration.

The tubular member with double cutting windows of the present invention described above may be part of an arthroscopic shaver employed in various surgical medical procedures such as conventional open surgeries or in other, less invasive, techniques that use cannulas or various port access devices. The present invention has applications in surgical procedures where the target tissue is ablated or shaped, and may be employed in cutting various body parts such as the knee, shoulder, hip, ankle, elbow, hand or foot. For example, the tubular member 100 of the present invention may be part of an arthroscopic shaver employed in arthroscopic surgery of a knee joint structure.

Although the present invention has been described in connection with preferred embodiments, many modifications and variations will become apparent to those skilled in the art. While preferred embodiments of the invention have been described and illustrated above, it should be understood that these are exemplary of the invention and are not to be considered as limiting. Accordingly, it is not intended that the present invention be limited to the illustrated embodiments, but only by the appended claims.

## Claims

1. An arthroscopic cutting instrument, comprising:
a tubular member comprising a closed distal end, and a cutting region located adjacent, and proximally, to the closed distal end; and
a first and second openings located on opposite sides of the cutting region of the tubular member, each of the first and second openings comprising two lateral cutting edges and corresponding first and second distal end edges, wherein the first and second distal end edges define the closed distal end.

2. The arthroscopic cutting instrument of claim 1, wherein the closed distal end is approximately perpendicular to a longitudinal axis of the tubular member.

3. The arthroscopic cutting instrument of claim 2, wherein the closed distal end has an arcuate configuration.

4. The arthroscopic cutting instrument of claim 1, wherein the first and second openings have an oval configuration.

5. The arthroscopic cutting instrument of claim 1, wherein the lateral cutting edges of the first opening has a first plurality of teeth, and the lateral cutting edges of the second opening has a second plurality of teeth.

6. The arthroscopic cutting instrument of claim 5, wherein the first and second plurality of teeth are symmetrically located relative to the longitudinal axis of the tubular member.

7. The arthroscopic cutting instrument of claim 5, wherein the first and second plurality of teeth are asymmetrically located relative to the longitudinal axis of the tubular member.

8. The arthroscopic cutting instrument of claim 5, wherein the first and second plurality of teeth have a pyramidal geometry.

9. The arthroscopic cutting instrument of claim 1, wherein the two lateral cutting edges of each opening converge to the closed distal end of the tubular member.

10. The arthroscopic cutting instrument of claim 1, wherein the first and second openings are cutting windows disposed asymmetrically relative to the longitudinal axis of the tubular member.

11. The arthroscopic cutting instrument of claim 1, wherein the first and second openings are cutting windows disposed symmetrically relative to the longitudinal axis of the tubular member.

12. The arthroscopic cutting instrument of claim 1, wherein the closed distal end has an arcuate configuration when the tubular member is viewed in an axial direction about parallel to the longitudinal axis of the tubular member.

13. The arthroscopic instrument of claim 1, wherein the closed distal end has a semicircular or a parabolic configuration when the tubular member is viewed in an axial direction about parallel to the longitudinal axis of the tubular member.

14. The arthroscopic cutting instrument of claim 1, wherein the instrument is a surgical shaver.

15. The arthroscopic cutting instrument of claim 1, wherein the lateral cutting edges of the first and second openings are created by a laser cutting process.
